(19) Europäisches Patentamt
European Patent Office
Office européen des brevets

(11) **EP 3 808 720 B1**

(12) **EUROPÄISCHE PATENTSCHRIFT**

(45) Veröffentlichungstag und Bekanntmachung des Hinweises auf die Patenterteilung:
**04.10.2023 Patentblatt 2023/40**

(21) Anmeldenummer: **19203314.0**

(22) Anmeldetag: **15.10.2019**

(51) Internationale Patentklassifikation (IPC):
***C07C 2/10*** *(2006.01)*

(52) Gemeinsame Patentklassifikation (CPC):
(C-Sets verfügbar)
**C07C 2/10;** C07C 2521/12; C07C 2523/755; C07C 2523/78 (Forts.)

(54) **VERFAHREN ZUR OLIGOMERISIERUNG VON OLEFINEN MIT STOFFSTRÖMEN MIT REDUZIERTEM OLEFINGEHALT**

METHOD FOR THE OLIGOMERISATION OF OLEFINS WITH FLOWS WITH REDUCED OLEFIN CONTENT

PROCÉDÉ D'OLIGOMÉRISATION D'OLÉFINES À L'AIDE DES FLUX DE MATIÈRE À CONTENU RÉDUIT D'OLÉFINES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.04.2021 Patentblatt 2021/16**

(73) Patentinhaber: **Evonik Operations GmbH 45128 Essen (DE)**

(72) Erfinder:
• **Stochniol, Guido 45721 Haltern am See (DE)**
• **Schallenberg, Jörg 46286 Dorsten (DE)**
• **Peitz, Stephan 45739 Oer-Erkenschwick (DE)**

(74) Vertreter: **Evonik Patent Association c/o Evonik Industries AG IP Management Bau 1042A/PB 15 Paul-Baumann-Straße 1 45772 Marl (DE)**

(56) Entgegenhaltungen:
**WO-A1-99/25668    WO-A1-2014/207034**

(52) Gemeinsame Patentklassifikation (CPC): (Forts.)

C-Sets
**C07C 2/10, C07C 11/02**

**Beschreibung**

Gegenstand der Erfindung ist ein Verfahren zur Oligomerisierung von C2- bis C8-Olefinen in mehreren Reaktionsstufen, bei dem das Einsatzgemisch und die jeweiligen Austräge aus den Reaktionsstufen aufgetrennt und zu verschiedenen Reaktionsstufen geführt werden.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

WO 2014/207034 A1 offenbart die Oligomerisierung von C4-Einsatzgemischen zu einem C8-Produktgemisch.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Verfahren zur Oligomerisierung von Olefinen sind im Stand der Technik hinlänglich bekannt und werden großindustriell eingesetzt. Die Produktionsmengen belaufen sich alleine in Deutschland auf mehrere tausend Kilotonnen pro Jahr. Um möglichst hohe Umsätze und einen möglichst kontinuierlichen Betrieb von Oligomerisierungsverfahren zu ermöglichen umfassen industrielle Anlagen meist nicht nur eine, sondern mindestens zwei hintereinandergeschaltete Reaktionsstufen. Dadurch kann das Oligomerisierungsverfahren selbst bei Ausfall einer Reaktionsstufe weiterbetrieben werden.

Die Quelle für die Olefine für die Oligomerisierungsverfahren sind in der Regel Steamcracker, in denen aus Naphtha kurzkettige Olefine wie Ethylen oder Propylen aber auch eine Butadien- und Buten-haltige C4-Fraktion (sogenanntes Crack-C4) gewonnen werden kann, die dann vom Isobuten befreit und dann einer Oligomerisierung zugeführt werden kann. Mittlerweile führt die Umstellung von Naphtha als Rohstoff für Steamcracker auf billigeres Ethan aus Shale-Gas dazu, dass der Anteil der Olefine in den erhaltenen Strömen geringer wird.

Niedrigere Olefinkonzentrationen können zur Weiterverarbeitung aber ein wirtschaftliches und technisches Problem darstellen. Bestehende Anlagen sind aufgrund ihres ursprünglichen Aufbaus meistens nicht darauf ausgelegt, auch bei niedrigen Olefinkonzentrationen ausreichend hohe Umsätze gewährleisten zu können. Außerdem können die integrierten Destillationskolonnen an ihre hydrodynamischen Grenzen stoßen, wenn der Anteil an nicht zu oligomerisierenden Inertanteilen, wie Alkanen, zu hoch wird. Zudem stellen die hohen Inertanteile einen negativen Kostenbeitrag dar, da diese Inertanteile sich energetisch ungünstig auf den Gesamtprozess auswirken.

Die Aufgabe der vorliegenden Erfindung war die Bereitstellung eines Verfahrens zur Oligomerisierung von Olefinen, welches die vorgenannten Probleme nicht aufweist. Die zugrundeliegende Aufgabe der vorliegenden Erfindung konnte mit dem Verfahren zur Oligomerisierung gemäß Anspruch 1 gelöst werden. Bevorzugte Ausgestaltungen sind in den Unteransprüchen angegeben.

Das erfindungsgemäße Verfahren ist ein Verfahren zur Oligomerisierung von C2- bis C8-Olefinen in mindestens drei hintereinandergeschalteten Reaktionsstufen, die jeweils mindestens einen Reaktor und mindestens eine Destillationskolonne umfassen, wobei das Verfahren die folgenden Schritte umfasst:

(a) Bereitstellen eines Einsatzgemisches, welches C2- bis C8-Olefine enthält, und Aufteilen des Einsatzgemisches in zwei Einsatzströme, wobei der eine Einsatzstrom als Zulaufstrom zur ersten Reaktionsstufe und der zweite Einsatzstrom als Zulaufstrom zu mindestens einer der nachfolgenden Reaktionsstufen, bei der der Olefingehalt weniger als 50 Gew.-% im Zulaufstrom beträgt, geführt wird;

(b) 1. Reaktionsstufe: Oligomerisierung der Olefine im Zulaufstrom zur ersten Reaktionsstufe in mindestens einem Reaktor unter Verwendung eines Oligomerisierungskatalysators und Abtrennen der dabei gebildeten Oligomere als Sumpfprodukt in einer nachfolgenden Destillationskolonne, wobei das in der Destillationskolonne gebildete Kopfprodukt zumindest teilweise als Zulaufstrom zur zweiten Reaktionsstufe geführt wird;

(c) 2. Reaktionsstufe: Oligomerisierung der Olefine im Zulaufstrom zur zweiten Reaktionsstufe in mindestens einem Reaktor unter Verwendung eines Oligomerisierungskatalysators und Abtrennen der dabei gebildeten Oligomere als Sumpfprodukt in einer Destillationskolonne, wobei das in der Destillationskolonne gebildete Kopfprodukt zumindest teilweise als Zulaufstrom zur dritten Reaktionsstufe geführt wird;

(d) 3. Reaktionsstufe: Oligomerisierung der Olefine im Zulaufstrom zur dritten Reaktionsstufe in mindestens einem Reaktor unter Verwendung eines Oligomerisierungskatalysators und Abtrennen der dabei gebildeten Oligomere als Sumpfprodukt in einer Destillationskolonne;

wobei der Reaktor oder die Reaktoren in der letzten Reaktionsstufe adiabatisch betrieben wird, aber die Reaktoren in den vorherigen Reaktionsstufen unter Verwendung eines Kühlmediums gekühlt werden; und wobei in den Reaktoren der einzelnen Reaktionsstufen ein Oligomerisierungskatalysator eingesetzt wird, welcher eine Nickelverbindung auf einem Alumosilicat-Trägermaterial umfasst und welcher weniger als 0,5 Gew.-% Titandioxid und Zirkoniumdioxid in seiner Gesamtzusammensetzung enthält wobei das Kopfprodukt aus der Destillationskolonne der letzten Reaktionsstufe vollständig aus dem Verfahren ausgeschleust wird.

Der Begriff "Reaktionsstufe" meint im Sinne der vorliegenden Erfindung einen Anlagenabschnitt, der einen oder mehreren Reaktor(en) und eine oder mehrere dem Reaktor nachfolgende Destillationskolonne(n) umfasst. In einer bevorzugten Ausführungsform ist nur eine Destillationskolonne pro Reaktionsstufe vorhanden. In den Destillationskolonnen werden insbesondere die erzeugten Oligomere von dem restlichen Ausgangsstrom aus dem Reaktor, welcher beispielsweise Alkane und nicht umgesetzte Olefine umfasst, getrennt. Übliche verfahrenstechnische Aggregate, die in den Reaktionsstufen eingebaut sein können, wie beispielsweise Vorwärmer für den Feed, Wärmetauscher oder ähnliches, werden hier nicht separat aufgeführt, sondern sind dem Fachmann geläufig.

Mit dem beschriebenen Ablauf, welches eine Kombination aus Stoffstromverteilung, variabler Reaktorbetriebsart und optimierter Prozessführung darstellt, konnte eine gegenüber einer konventionellen Fahrweise deutlich effizientere Nutzung der vorhandenen Olefine erreicht werden kann, insbesondere dann, wenn der Olefingehalt im Zulaufstrom einer der späteren Reaktionsstufen vergleichsweise gering ist. Ist der Olefingehalt im Zulaufstrom zu einer der späteren Reaktionsstufen aufgrund des Verbrauchs an Olefinen in der oder den vorherigen Reaktionstufe(n) zu gering, verschlechtern sich Umsätze und Raum-Zeit-Ausbeuten. Eine solche wirtschaftlich problematische Verfahrensführung kann durch das erfindungsgemäße Verfahren verhindert werden.

Das erfindungsgemäße Verfahren sieht aber nicht nur eine Aufteilung des in Schritt (a) bereitgestellten Einsatzgemisches vor, sondern auch die adiabatische Betriebsweise des Reaktors oder der Reaktoren in der letzten Reaktionsstufe. Die Formulierung "adiabtisch betrieben" ist so zu verstehen, dass der oder die Reaktor(en) in der letzten Reaktionsstufe nicht aktiv gekühlt werden. Die bei der Oligomerisierung freiwerdende Wärme wird stattdessen mit dem Produktstrom aus dem Reaktor geführt, wobei in der nachfolgenden Destillationskolonne weniger Energie zur Verdampfung benötigt wird und die Destillation damit energiesparender durchgeführt werden kann.

Das erfindungsgemäße Verfahren umfasst mindestens drei Reaktionsstufen. In einer bevorzugten Ausführungsform umfasst das Verfahren zur Oligomerisierung maximal fünf Reaktionsstufen. Insbesondere bevorzugt ist eine Verfahrensführung mit vier oder fünf Reaktionsstufen. Jede dieser Reaktionsstufen umfasst unabhängig voneinander einen oder mehreren Reaktoren und eine oder mehrere nachfolgende Destillationskolonnen, um die gebildeten Oligomere von dem restlichen Ausgangsstrom aus dem Reaktor abzutrennen. Es ist aber auch denkbar, dass eine der Reaktionsstufen mehrere Reaktoren umfasst, während in einer vorherigen oder nachfolgenden Reaktionsstufe nur ein Reaktor vorhanden ist.

Sind 4 Reaktionsstufen vorhanden wird das oben genannte erfindungsgemäße Verfahren mit einem geänderten Schritt (d) und einem zusätzlichen Schritt (e) wie folgt durchgeführt, wobei die Schritte (a) bis (c) wie beschrieben erhalten bleiben:

(d) 3. Reaktionsstufe: Oligomerisierung der Olefine im Zulaufstrom zur dritten Reaktionsstufe in mindestens einem Reaktor unter Verwendung eines Oligomerisierungskatalysators und Abtrennen der dabei gebildeten Oligomere als Sumpfprodukt in einer Destillationskolonne, wobei das in der Destillationskolonne gebildete Kopfprodukt zumindest teilweise als Zulaufstrom zur vierten Reaktionsstufe geführt wird; und

(e) 4. Reaktionsstufe: Oligomerisierung der Olefine im Zulaufstrom zur vierten Reaktionsstufe in mindestens einem Reaktor unter Verwendung eines Oligomerisierungskatalysators und Abtrennen der dabei gebildeten Oligomere als Sumpfprodukt in einer Destillationskolonne.

Sind 5 Reaktionsstufen vorhanden wird das vorgenannte erfindungsgemäße Verfahren mit 4 Reaktionsstufen mit einem geänderten Schritt (e) und einem zusätzlichen Schritt (f) wie folgt durchgeführt, wobei die Schritte (a) bis (d) wie beschrieben erhalten bleiben:

(e) 4. Reaktionsstufe: Oligomerisierung der Olefine im Zulaufstrom zur vierten Reaktionsstufe in mindestens einem Reaktor unter Verwendung eines Oligomerisierungskatalysators und Abtrennen der dabei gebildeten Oligomere als Sumpfprodukt in einer Destillationskolonne, wobei das in der Destillationskolonne gebildete Kopfprodukt zumindest teilweise als Zulaufstrom zur fünften Reaktionsstufe geführt wird; und

(f) 5. Reaktionsstufe: Oligomerisierung der Olefine im Zulaufstrom zur fünften Reaktionsstufe in mindestens einem Reaktor unter Verwendung eines Oligomerisierungskatalysators und Abtrennen der dabei gebildeten Oligomere als Sumpfprodukt in einer Destillationskolonne.

Das erfindungsgemäße Verfahren kann ganz allgemein wie folgt durchgeführt werden: Ausgangspunkt ist die Bereitstellung eines Einsatzgemisches, welches C2- bis C8-Olefine enthält. Das Einsatzgemisch wird vor dem Zulauf zur ersten Reaktionsstufe in zwei Einsatzströme aufgeteilt. Das Aufteilen kann in dem Fachmann bekannter Weise durchgeführt werden, beispielsweise über eine von der Hauptrohrleitung (für den ersten Einsatzstrom) abgehende Rohrleitung. Die Auftrennung der Ströme kann beispielsweise über eine Masseregelung erfolgen. Der eine (erste) Einsatzstrom wird als Zulaufstrom zur ersten Reaktionsstufe geleitet, wohingegen der andere (zweite) Einsatzstrom in Abhängigkeit vom Olefingehalt im Zulaufstrom zu einer der nachfolgenden Reaktionsstufen geleitet wird. Der Zulaufstrom der nachfolgenden Reaktionsstufen, also der zweiten und weiteren Reaktionsstufen, speist sich zumindest teilweise aus dem Kopfprodukt der Destillationskolonne der jeweils vorherigen Reaktionsstufe. Beträgt der Olefingehalt im Zulaufstrom zu einer der nachfolgenden Reaktionsstufen weniger als 50 Gew.-%, wird zusätzlich zumindest ein Teil des zweiten Einsatzstroms zudosiert, um den Anteil der Olefine in dem Zulaufstrom zu erhöhen.

In den einzelnen Reaktionsstufen wird der jeweilige Zulaufstrom erfindungsgemäß in dem mindestens einen Reaktor oligomerisiert und das erhaltene Oligomerisat jeweils zu einer Destillationskolonne geleitet, bei der die gebildeten Oligomere als Sumpfprodukt von dem restlichen Ausgangsstrom aus dem Reaktor, mindestens enthaltende Alkane und nicht umgesetzte Olefine, als Kopfprodukt getrennt. Je nach Reaktionsstufe wird das Kopfprodukt dann zumindest teilweise als Zulaufstrom zur jeweils nächsten Reaktionsstufe geleitet und optional teilweise zum Reaktor der jeweiligen Reaktionsstufe recycliert. In der letzten Reaktionsstufe, also der dritten, vierten, fünften oder nachfolgenden Reaktionsstufe, kann das Kopfprodukt der Destillationskolonne teilweise zum Reaktor in dieser Reaktionsstufe recycliert und teilweise aus dem Verfahren ausgeschleust werden. Wird das Kopfprodukt der Destillationskolonne der letzten Reaktionsstufe aus dem hier offenbarten Verfahren ausgeschleust, kann dies als Syntheserohstoff für weitere Verfahren (z. B. Hydroformylierung, C-Quelle für Lichtbogen bei der Acetylenherstellung), als Verbrennungsgas oder nach Vollhydrierung zu den Alkanen als Treibgas, als Kochgas o. ä. dienen.

Als Olefine für das erfindungsgemäße Verfahren werden C2- bis C8-Olefine, bevorzugt C2- bis C6-Olefine, weiterhin bevorzugt C3- bis C5-Olefine oder darauf basierende Olefingemische, die auch Anteile an analogen Alkanen enthalten können, eingesetzt. Geeignete Olefine sind unter anderem $\alpha$-Olefine, n-Olefine und Cycloalkene, vorzugsweise n-Olefine. In einer bevorzugten Ausführungsform handelt es sich bei dem Olefin um n-Buten.

Die Olefine werden üblicherweise nicht in reiner Form als Edukte eingesetzt, sondern in technisch verfügbaren Mischungen. Der in dieser Erfindung zusätzlich verwendete Begriff Einsatzgemisch ist deshalb so zu verstehend, dass er jegliche Art von Gemischen betrifft, die die entsprechenden zu oligomerisierenden Olefine in einer Menge enthalten, die es ermöglicht, die Oligomerisierung wirtschaftlich durchzuführen. Bevorzugt enthalten die erfindungsgemäß verwendeten Einsatzgemische praktisch keine weiteren ungesättigten Verbindungen und mehrfach ungesättigte Verbindungen wie Diene oder Acetylenderivate. Vorzugsweise werden Einsatzgemische eingesetzt, die weniger als 5 Gew.-%, insbesondere weniger als 2 Gew.-% an verzweigten Olefinen bezogen auf den Olefinanteil enthalten.

Propylen wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem $C_4$-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete Olefingemische sind insbesondere das Raffinat I und das Raffinat II und das Raffinat III.

Als Reaktor für die jeweiligen Reaktionsstufen können alle dem Fachmann bekannten Reaktoren eingesetzt werden, die für die Oligomerisierung geeignet sind, bspw. Rohrreaktoren, Rohrbündelreaktoren, Settler-Riser-Reaktoren, Slurry-Reaktoren. Bevorzugt sind Rohrreaktoren und/oder Rohrbündelreaktoren. Sofern eine Reaktionsstufe mehrere Reaktoren aufweist, können die Reaktoren gleich oder verschieden voneinander sein. Die Reaktoren in einer Reaktionsstufe können auch in ihrem Aufbau oder ihrer Ausgestaltung variieren. Der erste Reaktor in einer Reaktionsstufe kann beispielsweise ein größeres Volumen aufweisen als der nachfolgende Reaktor in der gleichen Reaktionsstufe. Ebenso ist es möglich, dass die Reaktoren in den einzelnen Reaktionsstufen gleich oder verschieden voneinander sein. Auch dabei ist es möglich, dass die Reaktoren in den einzelnen Reaktionsstufen in ihrem Aufbau oder ihrer Ausgestaltung unterschiedlich sind. Der Reaktor in der ersten Reaktionsstufe kann beispielsweise ein größeres Volumen aufweisen als ein oder alle Reaktoren in den nachfolgenden Reaktionsstufen.

Der eine oder die Reaktoren der einzelnen Reaktionsstufen enthalten jeweils einen Oligomerisierungskatalysator zur Durchführung der Oligomerisierung, insbesondere einen heterogenen Oligomerisierunsgkatalysator. Der Oligomerisierungskatalysator liegt dabei insbesondere in Form eines Granulats, eines Extrudats oder in Tablettenform vor.

Die (heterogenen) Oligomerisierungskatalysatoren umfassen eine Nickelverbindung, vorzugsweise Nickeloxid, auf einem Alumosilicat-Trägermaterial, enthalten aber weniger als 0,5 Gew.-%, bevorzugt weniger als 0,1 Gew.-%, besonders bevorzugt weniger als 0,01 Gew.-% Titandioxid und Zirkoniumdioxid bezogen auf die Gesamtzusammensetzung des Oligomerisierungskatalysators. Das Trägermaterial kann ein amorphes, mesoporöses Alumosilicat, ein kristallines, mikroporöses Alumosilicat oder eine Alumosilicat, welches amorphe und kristalline Phasen aufweist sein. Mit "amorph" im Sinne der vorliegenden Erfindung ist die Eigenschaft eines Feststoffes gemeint, die daraus folgt, dass der Feststoff im Gegensatz zu kristallinen Feststoffen keine Kristallstruktur, d. h. keine Fernordnung, aufweist.

Erfindungsgemäß weiterhin bevorzugt weist der Oligomerisierungskatalysator eine Zusammensetzung von 15 bis 40 Gew.-%, vorzugsweise 15 bis 30 Gew.-% NiO, 5 bis 30 Gew.-% $Al_2O_3$, 55 bis 80 Gew.-% $SiO_2$ und 0,01 bis 2,5 Gew.-%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. Der Oligomerisierungskatalysator ist im Wesentlichen frei von Titandioxid und Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und Zirkoniumdioxid in seiner Gesamtzusammensetzung.

Der Oligomerisierungskatalysator weist vorzugsweise eine spezifische Oberfläche (berechnet nach BET) von 150 bis 700 m$^2$/g, weiterhin bevorzugt von 190 bis 600 m$^2$/g, besonders bevorzugt von 220 bis 550 m$^2$/g aufweisen. Die BET-Oberfläche wird mittels Stickstoff-Physisorption gemäß DIN-ISO 9277 (Stand: 2014-01) gemessen.

Die in den einzelnen Reaktoren in den Reaktionsstufen vorhandenen Oligomerisierungskatalysatoren können jeweils unabhängig voneinander aus den vorgenannten Substanzen ausgewählt werden. Die einzelnen Oligomerisierungskatalysatoren in den Reaktoren sind dabei nicht immer exakt identisch, sondern unterscheiden sich in der Zusammensetzung voneinander, möglicherweise auch nur in geringem Umfang. Dies liegt auch daran, dass selbst wenn zum Zeitpunkt der ersten Inbetriebnahme des erfindungsgemäßen Verfahrens jeder Reaktor eine völlig identische Katalysatorzusammensetzung enthält, sich diese Zusammensetzung während des Betriebs mit der Zeit durch verschiedenste Effekte im Laufe der Jahre (Regenerierter Katalysator verhält sich anders als neu hergestellte Katalysatoren, Abrieb während des Betriebes, unterschiedlich schnelle Alterung und/oder Vergiftung, etc.) ändern.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden.

Die Oligomerisierung kann in jeder der vorhandenen Reaktionsstufen bei einer Temperatur im Bereich von 50 bis 200 °C, vorzugsweise 60 bis 180 °C, bevorzugt im Bereich von 60 bis 130°C durchgeführt wird. Der Druck kann jeder der vorhandenen Reaktionsstufen von 10 bis 70 bar, bevorzugt von 20 bis 55 bar betragen. In einer bevorzugten Ausführungsform der vorliegenden Erfindung wird die Oligomerisierung in jeder Reaktionsstufe in flüssiger Phase durchgeführt. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt.

Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (= 1 h$^{-1}$) und 190 h$^{-1}$, vorzugsweise zwischen 2 h$^{-1}$ und 35 h$^{-1}$, besonders bevorzugt zwischen 3 h$^{-1}$ und 25 h$^{-1}$.

Insbesondere bei Verwendung eines Katalysators, welcher eine Nickelverbindung, vorzugsweise Nickeloxid, auf einem Alumosilicat-Trägermaterial umfasst, beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Oligomerisierung von Olefinen ist eine exotherme Reaktion, also eine Reaktion, bei der Wärme freigesetzt wird. Um die Oligomerisierungstemperatur in einem gewünschten Bereich zu halten, können die Reaktoren gekühlt werden, um einen Teil der oder die gesamte freigeworde Wärme abzuführen. Um die freigeworde Wärme für nachfolgende Prozesse zu nutzen, kann auf eine Kühlung teilweise vollständig verzichtet werden, so wie es in der letzten Reaktionsstufe der Fall ist. Die bei der Oligomerisierung freiwerdende Wärme wird durch den Austrag des Produktstroms aus dem Reaktor abgeführt und in der Destillationskolonne insofern genutzt, als dass weniger Energie bereitgestellt werden muss, um die gewünschte Trennwirkung bei der Destillation zu erreichen.

In einer bevorzugten Ausführungsform werden bis auf die letzte Reaktionsstufe alle Reaktoren in den vorherigen Reaktionsstufen gekühlt. Dabei kann ein dem Fachmann bekanntes Kühlmedium, beispielsweise Kühlwasser, eingesetzt werden. In einer bevorzugten Ausführungsform sollte der Temperaturanstieg im Reaktor trotz Kühlens 5 K nicht überschreiten. Dies entspricht einer isothermen Betriebsweise der Reaktoren. Bezogen auf eine Kühlleistung von 100% für den oder die Reaktor(en) in der ersten Reaktionsstufe, beträgt die Kühlleistung in dem oder den (Reaktor(en) der nachfolgenden Reaktionsstufen weniger als 100%, aber außer in der letzten Reaktionsstufe nicht 0%.

In einer ganz bevorzugten Ausführungsform, bei Vorhandensein von drei Reaktionsstufen, beträgt die Kühlleistung für den oder die Reaktor(en) der ersten Reaktionsstufe 100% und für den oder die Reaktor(en) der zweiten Reaktionsstufe 10 bis 60%, wobei der Reaktor der dritten und letzten Reaktionsstufe adiabatisch betrieben wird. In einer weiteren ganz bevorzugten Ausführungsform, bei Vorhandensein von vier Reaktionsstufen, beträgt die Kühlleistung für den oder die Reaktor(en) der ersten Reaktionsstufe 100%, für den oder die Reaktor(en) der zweiten Reaktionsstufe 40 bis 60% und für den oder die Reaktor(en) der dritten Reaktionsstufe 10 bis 30%, wobei der Reaktor der vierten und letzten Reaktionsstufe adiabatisch betrieben wird.

Die bei der Kühlung in den der adiabat betriebenen vorhergehenden Reaktionsstufen durch das Kühlmedium aufgenommene Wärme kann in einer bevorzugten Ausführungsform genutzt werden, um einen oder mehrere der Zulaufströme, bevorzugt alle Zulaufströme zu den einzelnen Reaktionsstufen aufzuwärmen, vorzugsweise auf einen Temperatur T > 50 °C. Dies kann in dem Fachmann bekannter Weise durchgeführt werden, insbesondere durch die Verwendung eines Wärmetauschers. So kann die bei der Reaktion entstehende und über das Kühlmedium bei der Kühlung aufgenommene Wärme noch für das weitere Verfahren verwendet werden, was aus wirtschaftlicher und ökologischer Sicht vorteilhaft ist.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel, wobei sich der Anteil der einzelnen Dimerfraktionen auf die Gesamt-dimer-Fraktion bezieht:

$$\frac{(\text{einfach verzweigte Dimere (Gew.-\%)} + 2 \times \text{zweifach verzweigte Dimere (Gew.-\%)})}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das $C_9$-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

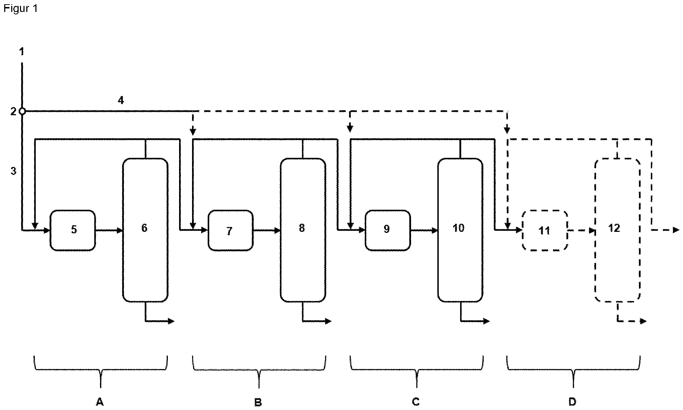

Figur 1 zeigt eine Ausführungsform entsprechend der vorliegenden Erfindung. Der Einsatzstrom (1) wird über wird zunächst in den Zulaufstrom zur ersten Reaktionsstufe (3) und den Zulaufstrom zu mindestens einer der nachfolgenden Reaktionsstufen mit einem Olefingehalt von weniger als 50% (4) am Punkt (2) aufgeteilt. Der Zulaufstrom (3) wird dann zum Reaktor (5) der ersten Reaktionsstufe (A) und das aus dem Reaktor erhaltene Oligomerisat zur Destillationskolonne (6) geführt, wo die gebildeten Oligomere als Sumpfprodukt abgetrennt werden. Das Kopfprodukt wird teilweise zurück zum Reaktor (5) und teilweise zur nächsten, zweiten Reaktionsstufe (B) geführt. Gegebenenfalls, d.h. wenn der Olefingehalt in der zweiten Stufe bereits unter 50% im Zulauf liegt, kann der vom Einsatzstrom abgetrennte Einsatzstrom (4) bereits zur zweiten Reaktionsstufe (B) zudosiert werden. Dort erfolgt zunächst eine Oligomerisierung in einem Reaktor (7). Das erhaltene Oligomerisat gelangt zur Destillationskolonne (8), wo die im Reaktor (7) der zweiten Reaktionsstufe (B) gebildeten Oligomere über den Sumpf abgetrennt werden. Das Kopfprodukt wird teilweise zum Reaktor (7) und teilweise zur dritten Reaktionsstufe (C) geführt. Dort wird der Zulaufstrom einer Oligomerisierung im Reaktor (9) unterworfen und die Oligomere als Sumpfprodukt in der Destillationskolonne (10) abgetrennt. Ein Teil des Kopfprodukts wird zum Reaktor (9) recycliert. Der vom Einsatzstrom abgetrennte Zulaufstrom (4) kann hier zusätzlich zum recyclierten Kopfprodukt zugeführt werden (gestrichelter Pfeil). Die vierte Reaktionsstufe (D) ist optional und deshalb gestrichelt dargestellt. Die optionale vierte Reaktionsstufe umfasst ebenfalls einen Reaktor (11) und eine Destillationskolonne (12). Der Zulaufstrom kann in der Ausführungsform mit 4 Reaktionsstufen zur dritten und/oder vierten Reaktionsstufe geführt werden. Die Ausführungsform mit 5 Reaktionsstufen ist in Figur 1 nicht enthalten, der gezeigte Aufbau wird dann jedoch leidglich eine weitere Reaktionsstufe nach Reaktionsstufe (D) umfassen.

**Patentansprüche**

1. Verfahren zur Oligomerisierung von C2- bis C8-Olefinen in mindestens drei hintereinandergeschalteten Reaktionsstufen, die jeweils mindestens einen Reaktor und mindestens eine Destillationskolonne umfassen, wobei das Verfahren die folgenden Schritte umfasst:

   (a) Bereitstellen eines Einsatzgemisches, welches C2- bis C8-Olefine enthält, und Aufteilen des Einsatzgemisches in zwei Einsatzströme, wobei der eine Einsatzstrom als Zulaufstrom zur ersten Reaktionsstufe und der zweite Einsatzstrom als Zulaufstrom zu mindestens einer der nachfolgenden Reaktionsstufe, bei der der Olefingehalt weniger als 50 Gew.-% im Zulaufstrom beträgt, geführt wird;
   (b) 1. Reaktionsstufe: Oligomerisierung der Olefine im Zulaufstrom zur ersten Reaktionsstufe in mindestens einem Reaktor unter Verwendung eines Oligomerisierungskatalysators und Abtrennen der dabei gebildeten Oligomere als Sumpfprodukt in einer nachfolgenden Destillationskolonne, wobei das in der Destillationskolonne gebildete Kopfprodukt zumindest teilweise als Zulaufstrom zur zweiten Reaktionsstufe geführt wird;
   (c) 2. Reaktionsstufe: Oligomerisierung der Olefine im Zulaufstrom zur zweiten Reaktionsstufe in mindestens einem Reaktor unter Verwendung eines Oligomerisierungskatalysators und Abtrennen der dabei gebildeten Oligomere als Sumpfprodukt in einer Destillationskolonne, wobei das in der Destillationskolonne gebildete Kopfprodukt zumindest teilweise als Zulaufstrom zur dritten Reaktionsstufe geführt wird;
   (d) 3. Reaktionsstufe: Oligomerisierung der Olefine im Zulaufstrom zur dritten Reaktionsstufe in mindestens einem Reaktor unter Verwendung eines Oligomerisierungskatalysators und Abtrennen der dabei gebildeten Oligomere als Sumpfprodukt in einer Destillationskolonne;
   wobei der Reaktor oder die Reaktoren in der letzten Reaktionsstufe adiabatisch betrieben wird, aber die Reaktoren in den vorherigen Reaktionsstufen unter Verwendung eines Kühlmediums gekühlt werden; und
   wobei in den Reaktoren der einzelnen Reaktionsstufen ein Oligomerisierungskatalysator eingesetzt wird, welcher eine Nickelverbindung auf einem Alumosilicat-Trägermaterial umfasst und welcher weniger als 0,5 Gew.-% Titandioxid und Zirkoniumdioxid in seiner Gesamtzusammensetzung enthält
   wobei das Kopfprodukt aus der Destillationskolonne der letzten Reaktionsstufe vollständig aus dem Verfahren ausgeschleust wird.

2. Verfahren zur Oligomerisierung nach Anspruch 1, wobei bezogen auf eine Kühlleistung von 100% für den oder die Reaktor(en) in der ersten Reaktionsstufe die Kühlleistung in dem oder den (Reaktor(en) der nachfolgenden Reaktionsstufen weniger als 100%, aber nur in der letzten Reaktionsstufe 0% beträgt.

3. Verfahren zur Oligomerisierung nach Anspruch 1 oder 2, wobei die durch das Kühlmedium aufgenommene Wärme genutzt wird, um einen oder mehrere der Zulaufströme zu den einzelnen Reaktionsstufen aufzuwärmen.

4. Verfahren zur Oligomerisierung nach Anspruch 3, wobei die aufgewärmten Zulaufströme zu den einzelnen Reaktionsstufen eine Temperatur von > 50 °C aufweisen.

5. Verfahren zur Oligomerisierung nach einem der Ansprüche 1 bis 4, wobei die Oligomerisierung in jeder der vorhandenen Reaktionsstufen bei einer Temperatur im Bereich von 50 bis 200 °C, vorzugsweise 60 bis 180 °C, besonders bevorzugt im Bereich von 60 bis 130°C durchgeführt wird.

6. Verfahren zur Oligomerisierung nach einem der Ansprüche 1 bis 5, wobei der Druck bei der Oligomerisierung jeder der vorhandenen Reaktionsstufen von 10 bis 70 bar, bevorzugt von 20 bis 55 bar beträgt.

7. Verfahren zur Oligomerisierung nach einem der Ansprüche 1 bis 6, wobei die Oligomerisierungskatalysatoren in den Reaktoren der einzelnen Reaktionsstufen eine Zusammensetzung von 15 bis 40 Gew.-% NiO, 5 bis 30 Gew.-% $Al_2O_3$, 55 bis 80 Gew.-% $SiO_2$ und 0,01 bis 2,5 Gew.-% eines Alkalimetalloxids aufweist.

8. Verfahren zur Oligomerisierung einem der Ansprüche 1 bis 7, wobei das Verfahren 4 Reaktionsstufen und bezogen auf Anspruch 1 einen geänderten Schritt (d) und einen zusätzlichen Schritt (e) aufweist:

   (d) 3. Reaktionsstufe: Oligomerisierung der Olefine im Zulaufstrom zur dritten Reaktionsstufe in mindestens einem Reaktor unter Verwendung eines Oligomerisierungskatalysators und Abtrennen der dabei gebildeten Oligomere als Sumpfprodukt in einer Destillationskolonne, wobei das in der Destillationskolonne gebildete Kopfprodukt zumindest teilweise als Zulaufstrom zur vierten Reaktionsstufe geführt wird; und
   (e) 4. Reaktionsstufe: Oligomerisierung der Olefine im Zulaufstrom zur vierten Reaktionsstufe in mindestens einem Reaktor unter Verwendung eines Oligomerisierungskatalysators und Abtrennen der dabei gebildeten Oligomere als Sumpfprodukt in einer Destillationskolonne.

9. Verfahren zur Oligomerisierung nach Anspruch 8, wobei das Verfahren 5 Reaktionsstufen und bezogen auf Anspruch 8 einen geänderten Schritt (e) und einen zusätzlichen Schritt (f) aufweist:

(e) 4. Reaktionsstufe: Oligomerisierung der Olefine im Zulaufstrom zur vierten Reaktionsstufe in mindestens einem Reaktor unter Verwendung eines Oligomerisierungskatalysators und Abtrennen der dabei gebildeten Oligomere als Sumpfprodukt in einer Destillationskolonne, wobei das in der Destillationskolonne gebildete Kopfprodukt zumindest teilweise als Zulaufstrom zur fünften Reaktionsstufe geführt wird; und
(f) 5. Reaktionsstufe: Oligomerisierung der Olefine im Zulaufstrom zur fünften Reaktionsstufe in mindestens einem Reaktor unter Verwendung eines Oligomerisierungskatalysators und Abtrennen der dabei gebildeten Oligomere als Sumpfprodukt in einer Destillationskolonne.

## Claims

1. Process for oligomerizing C2- to C8-olefins in at least three reaction stages connected in series, each comprising at least one reactor and at least one distillation column, wherein the method comprises the following steps:

(a) providing a starting mixture comprising C2- to C8-olefins, and dividing the starting mixture into two feed streams, wherein the one feed stream is fed as feed stream to the first reaction stage and the second feed stream is fed as feed stream to at least one of the downstream reaction stages in which the olefin content in the feed stream is less than 50% by weight;
(b) 1st reaction stage: oligomerization, using an oligomerization catalyst, of the olefins in the feed stream to the first reaction stage in at least one reactor and separating the oligomers formed in this case as bottom product in a downstream distillation column, wherein the overhead product formed in the distillation column is at least partially fed as feed stream to the second reaction stage;
(c) 2nd reaction stage: oligomerization, using an oligomerization catalyst, of the olefins in the feed stream to the second reaction stage in at least one reactor and separating the oligomers formed in this case as bottom product in a distillation column, wherein the overhead product formed in the distillation column is at least partially fed as feed stream to the third reaction stage;
(d) 3rd reaction stage: oligomerization, using an oligomerization catalyst, of the olefins in the feed stream to the third reaction stage in at least one reactor and separating the oligomers formed in this case as bottom product in a distillation column;
wherein the reactor(s) in the last reaction stage are operated adiabatically, but the reactors in the preceding reaction stages are cooled using a cooling medium; and
wherein an oligomerization catalyst is used in the reactors of the individual reaction stages comprising a nickel compound on an aluminosilicate support material and which comprises less than 0.5% by weight titanium dioxide and zirconium dioxide in its overall composition,
wherein the overhead product of the distillation column of the last reaction stage is fully discharged from the process.

2. Process for oligomerization according to Claim 1, wherein, based on a cooling power of 100% for the reactor(s) in the first reaction stage, the cooling power in the reactor(s) of the subsequent reaction stages is less than 100% but is 0% only in the last reaction stage.

3. Process for oligomerization according to Claim 1 or 2, wherein the heat absorbed by the cooling medium is used to heat one or more of the feed streams to the individual reaction stages.

4. Process for oligomerization according to Claim 3, wherein the heated feed streams to the individual reaction stages have a temperature of > 50°C.

5. Process for oligomerization according to any of Claims 1 to 4, wherein the oligomerization in each of the reaction stages present is carried out at a temperature in the range of 50 to 200°C, preferably 60 to 180°C, particularly preferably in the range of 60 to 130°C.

6. Process for oligomerization according to any of Claims 1 to 5, wherein the pressure in the oligomerization of each of the reaction stages present is 10 to 70 bar, preferably 20 to 55 bar.

7. Process for oligomerization according to any of Claims 1 to 6, wherein the oligomerization catalyst in the reactors of the individual reaction stages has a composition of 15 to 40% by weight NiO, 5 to 30% by weight $Al_2O_3$, 55 to 80% by weight $SiO_2$ and 0.01 to 2.5% by weight of an alkali metal oxide.

8. Process for oligomerization according to any of Claims 1 to 7, wherein the process has 4 reaction stages and, based on Claim 1, a modified step (d) and an additional step (e):

   (d) 3rd reaction stage: oligomerization, using an oligomerization catalyst, of the olefins in the feed stream to the third reaction stage in at least one reactor and separating the oligomers formed in this case as bottom product in a distillation column, wherein the overhead product formed in the distillation column is at least partially fed as feed stream to the fourth reaction stage; and
   (e) 4th reaction stage: oligomerization, using an oligomerization catalyst, of the olefins in the feed stream to the fourth reaction stage in at least one reactor and separating the oligomers formed in this case as bottom product in a distillation column.

9. Process for oligomerization according to Claim 8, wherein the process has five reaction stages and, based on Claim 8, a modified step (e) and an additional step (f):

   (e) 4th reaction stage: oligomerization, using an oligomerization catalyst, of the olefins in the feed stream to the fourth reaction stage in at least one reactor and separating the oligomers formed in this case as bottom product in a distillation column, wherein the overhead product formed in the distillation column is at least partially fed as feed stream to the fifth reaction stage; and
   (f) 5th reaction stage: oligomerization, using an oligomerization catalyst, of the olefins in the feed stream to the fifth reaction stage in at least one reactor and separating the oligomers formed in this case as bottom product in a distillation column.

## Revendications

1. Procédé d'oligomérisation d'oléfines en C2 à C8 dans au moins trois étages de réactions successifs, qui comprennent à chaque fois au moins un réacteur et au moins une colonne de distillation, le procédé comprenant les étapes suivantes :

   (a) fourniture d'un mélange de départ, qui contient des oléfines en C2 à C8, et répartition du mélange de départ en deux flux de départ, l'un flux de départ étant guidé en tant que flux d'alimentation vers le premier étage de réaction et le deuxième flux de départ étant guidé en tant que flux d'alimentation vers au moins l'un des étages de réaction consécutifs, dans lequel la teneur en oléfines dans le flux d'alimentation est inférieure à 50% en poids ;
   (b) 1er étage de réaction : oligomérisation des oléfines dans le flux d'alimentation vers le premier étage de réaction dans au moins un réacteur à l'aide d'un catalyseur d'oligomérisation et séparation des oligomères ainsi formés en tant que produit de fond dans une colonne de distillation consécutive, le produit de tête formé dans la colonne de distillation étant guidé au moins partiellement en tant que flux d'alimentation vers le deuxième étage de réaction ;
   (c) 2ème étage de réaction : oligomérisation des oléfines dans le flux d'alimentation vers le deuxième étage de réaction dans au moins un réacteur à l'aide d'un catalyseur d'oligomérisation et séparation des oligomères ainsi formés en tant que produit de fond dans une colonne de distillation, le produit de tête formé dans la colonne de distillation étant guidé au moins partiellement en tant que flux d'alimentation vers le troisième étage de réaction ;
   (d) 3ème étage de réaction : oligomérisation des oléfines dans le flux d'alimentation vers le troisième étage de réaction dans au moins un réacteur à l'aide d'un catalyseur d'oligomérisation et séparation des oligomères ainsi formés en tant que produit de fond dans une colonne de distillation ;
   le réacteur ou les réacteurs dans le dernier étage de réaction étant exploité (s) de manière adiabatique mais les réacteurs dans les étages réaction précédents étant refroidis à l'aide d'un agent caloporteur ; et
   un catalyseur d'oligomérisation, qui comprend un composé de nickel sur un matériau support d'aluminosilicate et qui contient moins de 0,5% en poids de dioxyde de titane et de dioxyde de zirconium dans sa composition globale, étant utilisé dans les réacteurs des différents étages de réaction ;
   le produit de tête étant soutiré complètement du procédé à partir de la colonne de distillation du dernier étage de réaction.

2. Procédé d'oligomérisation selon la revendication 1, la puissance de refroidissement dans le(s) réacteur(s) des étages de réaction consécutifs, par rapport à une puissance de refroidissement de 100% pour le(s) réacteur(s) dans le premier étage de réaction, étant inférieure à 100%, mais ne valant 0% que dans le dernier étage de réaction.

3. Procédé d'oligomérisation selon la revendication 1 ou 2, la chaleur absorbée par l'agent caloporteur étant utilisée pour réchauffer un ou plusieurs des flux d'alimentation vers les différents étages de réaction.

4. Procédé d'oligomérisation selon la revendication 3, les flux d'alimentation vers les différents étages de réaction, réchauffés, présentant une température > 50°C.

5. Procédé d'oligomérisation selon l'une quelconque des revendications 1 à 4, l'oligomérisation dans chacun des étages de réaction présents étant réalisée à une température dans la plage de 50 à 200°C, de préférence de 60 à 180°C, de manière particulièrement préférée dans la plage de 60 à 130°C.

6. Procédé d'oligomérisation selon l'une quelconque des revendications 1 à 5, la pression lors de l'oligomérisation dans chacun des étages de réaction présents étant de 10 à 70 bars, de préférence de 20 à 55 bars.

7. Procédé d'oligomérisation selon l'une quelconque des revendications 1 à 6, les catalyseurs d'oligomérisation dans les réacteurs des différents étages de réaction présentant une composition de 15 à 40% en poids de NiO, 5 à 30% en poids de $Al_2O_3$, 55 à 80% en poids de $SiO_2$ et 0,01 à 2,5% en poids d'un oxyde de métal alcalin.

8. Procédé d'oligomérisation selon l'une quelconque des revendications 1 à 7, le procédé présentant 4 étages de réaction et présentant, par rapport à la revendication 1, une étape (d) modifiée et une étape (e) supplémentaire :

   (d) 3ème étage de réaction : oligomérisation des oléfines dans le flux d'alimentation vers le troisième étage de réaction dans au moins un réacteur à l'aide d'un catalyseur d'oligomérisation et séparation des oligomères ainsi formés en tant que produit de fond dans une colonne de distillation, le produit de tête formé dans la colonne de distillation étant guidé au moins partiellement en tant que flux d'alimentation vers le quatrième étage de réaction ;
   (e) 4ème étage de réaction : oligomérisation des oléfines dans le flux d'alimentation vers le quatrième étage de réaction dans au moins un réacteur à l'aide d'un catalyseur d'oligomérisation et séparation des oligomères ainsi formés en tant que produit de fond dans une colonne de distillation.

9. Procédé d'oligomérisation selon la revendication 8, le procédé présentant 5 étages de réaction et présentant, par rapport à la revendication 8, une étape (e) modifiée et une étape (f) supplémentaire :

   (e) 4ème étage de réaction : oligomérisation des oléfines dans le flux d'alimentation vers le quatrième étage de réaction dans au moins un réacteur à l'aide d'un catalyseur d'oligomérisation et séparation des oligomères ainsi formés en tant que produit de fond dans une colonne de distillation, le produit de tête formé dans la colonne de distillation étant guidé au moins partiellement en tant que flux d'alimentation vers le cinquième étage de réaction ;
   (f) 5ème étage de réaction : oligomérisation des oléfines dans le flux d'alimentation vers le cinquième étage de réaction dans au moins un réacteur à l'aide d'un catalyseur d'oligomérisation et séparation des oligomères ainsi formés en tant que produit de fond dans une colonne de distillation.

Figur 1

**EP 3 808 720 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014207034 A1